# EUROPÄISCHE PATENTSCHRIFT

(11) **EP 0 927 036 B1**
(45) Veröffentlichungstag und Bekanntmachung des Hinweises auf die Patenterteilung: **02.03.2005**
(21) Anmeldenummer: 97937573.0
(22) Anmeldetag: 13.08.1997
(51) Int. Cl.: A61K 31/565, A61K 31/66, A61K 31/00, A61K 9/70

(54) **TRANSDERMALES THERAPEUTISCHES SYSTEM MIT EINER ÖSTRIOL ENTHALTENDEN BISPHOSPHATEKOMBINATION**
TRANSDERMAL THERAPEUTICAL APPROACH INVOLVING A COMBINATION OF BISPHOSPHATE CONTAINING OESTRIOL
SYSTEME THERAPEUTIQUE TRANSDERMIQUE METTANT EN OEUVRE UNE COMBINAISON DE BISPHOSPHATE A TENEUR EN OSTRIOL

(30) Priorität: 04.09.1996 DE 19635883
(43) Veröffentlichungstag der Anmeldung: 07.07.1999
(73) Patentinhaber: LTS LOHMANN Therapie-Systeme AG, 56626 Andernach (DE)
(72) Erfinder: VON KLEINSORGEN, Reinhard, D-56170 Bendorf (DE); VON KLEINSORGEN, Britta, D-56170 Bendorf (DE)
(74) Vertreter: Flaccus, Rolf-Dieter, Dr.
(86) Internationale Anmeldenummer: PCT/EP1997/004392
(87) Internationale Veröffentlichungsnummer: WO 1998/009631

(56) Entgegenhaltungen:
- WO-A-92/14474
- WO-A-93/00058
- WO-A-93/18774
- WO-A-95/16440

## Beschreibung

Die Erfindung betrifft ein transdermales therapeutisches System mit einer Östriol enthaltenden Wirkstoffkombination.

Östrogene sind Steroidhormone, die sich von dem tetrazyklischen C₁₈-Steroid Östran ableiten. Unter den natürlichen Östrogenen unterscheidet man Östron, Östradiol und Östriol, wobei Östron und Östriol als die physiologisch wichtigsten gelten.
Östriol ist eines der metabolischen Endprodukte des Estradiolstoffwechsels.
Es besitzt eine Reihe pharmakologischer und biologischer Besonderheiten, die es von anderen Östrogenen unterscheidet:
Es wird schon vor der Absorption fast vollständig konjugiert. Nur etwa 1 - 2 % des eingenommenen Östriols erscheinen als freies Östriol im Kreislauf. Der Quotient aus freiem zu konjugiertem Östriol beträgt 1 : 500.
Es übt bei einer oralen Dosis von 2 mg keine proliferierende Wirkung auf das Endometrium aus, da es nur kurze Zeit an die Rezeptoren des Zellkerns gebunden bleibt. Östrogene Wirkungen werden aber nur dann ausgelöst, wenn eine östrogene Substanz eine längere Zeitspanne im Kern verbleibt. Dies ist mit Östriol nur möglich, wenn es mehrmals am Tage verabreicht wird.
Eine weitere Besonderheit des Östriols besteht darin, daß bei mehrmonatiger Östrioleinnahme die östrogene Wirkung zunimmt.
Nach vaginaler Applikation liegt der Anteil von unkonjugiertem Östriol im Serum 10 bis 20 mal höher als nach oraler Gabe, da die intestinale Metabolisierung entfällt. Bei einer Östrioldosis von 0,5 mg kann man bereits 2 Stunden nach vaginaler Applikation mit einem hohen Serumspiegel von 100-150 pg/ml rechnen . Für den gleichen Effekt benötigt man dagegen eine orale Dosis von 10 mg.

Die obigen Erfahrungen lassen den Schluß zu, daß für Östriol ein transdermales therapeutisches System (TTS) galenisch das System der Wahl darstellt. Durch das System kann sichergestellt werden, daß Östriol über einen Zeitraum von z.B. 7 Tagen kontinuierlich an den Organismus abgegeben wird.

Dem Wirkstoff Östriol wurde bisher eine unzureichende therapeutische Wirksamkeit im Rahmen der Substitutionstherapie (HRT) zugesprochen. Dies gilt in besonderem Maße für die Verwendung von Östriol zur Prävention der Osteoporose. So wird in einer offiziellen Stellungnahme der Deutschen Gesellschaft Endokrinologie die Unwirksamkeit von Östriol zu Osteoporose Prophylaxe ausdrücklich hervorgehoben (vgl. Deutsches Ärzteblatt - Ärztliche Mitteilungen, 85, 1322-1325, (1988).

Die Unwirksamkeit von Östriol am Knochen ist inzwischen als Standardwissen in die einschlägigen Lehrbücher eingegangen (Freimut A. Leidenberger, "Klinische Endokrinologie für Frauenärzte " Springer Verlag 1992, Seite 356).

Auf die Unwirksamkeit von Östriol allein für die Behandlung von Osteoporose wird ferner auch in Produktinformationen für Präparate hingewiesen, die Östriol als Wirkstoff enthalten (z.B. Jenapharm Arzneimittel: Sortiment und Preise vom 01.07.1991 S.67).

Im Gegensatz hierzu wird in der PCT-Anmeldung WO 93/18774 die alleinige Verwendung von Östriol zur Behandlung von Osteoporose in Form eines transdermalen therapeutischen Systems dargestellt. Danach sprechen die Befunde für Östriol als das Östrogen der Wahl für die kontinuierliche Hormon-Substitutions-Therapie und insbesondere für die Therapie klimakterischer Osteoporose. Bei kontinuierlicher Verabreichung wird nämlich einerseits Osteoporose wirksam therapiert bzw. präventiert, während andererseits die bei herkömmlichen Östrogenen beobachtete kanzerogene Wirkung entfällt und sogar eine antikanzerogene Wirkung erwartet werden kann.

WO 92/14474 beschreibt die Verwendung einer Östradiol enthaltenden Biophosphonate Kombination.

Ausgehend von diesem Stand der medizinischen Wissenschaft liegt der Erfindung die Aufgabe zugrunde, ein wesentlich verbessertes transdermales therapeutisches System mit einer Östriol enthaltenden Wirkstoffkombination anzugeben, die ohne Risiken und schädliche Nebenwirkungen eine besonders hohe therapeutische Wirksamkeit und Akzeptanz bei der Verwendung von Östriol für die Prävention von Osteoporose im Alter entfaltet.

Überraschenderweise wurde gefunden, daß transdermal appliziertes Östriol die Wirkung von transdermal applizierten Biphosphonaten mit Biphosphonaten eignet es sich bei transdermaler Applikation zur Behandlung von Osteoporose.

Bei weiteren Anwendungen wurde gefunden, daß in der Behandlung der Osteoporose die transdermale Applikation von Biphosphonaten in Kombination mit Östriol vorteilhafter ist als Östriol allein. Eine bevorzugte Dosierungsform ist eine solche, welche in 24 Stunden 8 bis 16 mg Östriol und 3 bis 7 mg Biphosphonat pro TTS freisetzt.

## Patentansprüche

1. Transdermales therapeutisches System zur Behandlung von Osteoporose, **dadurch gekennzeichnet, daß** es Östriol in Kombination mit mindestens einem Wirkstoff aus der Klasse der Bisphosphonate enthält.

2. Transdermales therapeutisches System nach Anspruch 1, **dadurch gekennzeichnet, daß** es innerhalb von 24 Stunden 8 bis 16 mg Östriol und 3 bis 7 mg Bisphosphonat freisetzt.

3. Verwendung von Östriol in Kombination mit mindestens einem Wirkstoff aus der Gruppe der Bisphosphonate zur Herstellung eines transdermalen therapeutischen Systems zur Behandlung der Osteoporose.

## Claims

1. Transdermal therapeutic system for treating osteoporosis, **characterized in that** it contains estriol in combination with at least one active substance from the class of the bisphosphonates.

2. Transdermal therapeutic system according to claim 1, **characterized in that** it releases 8 to 16 mg of estriol and 3 to 7 mg of bisphosphonate within 24 hours.

3. Use of estriol in combination with at least one active substance from the group of the bisphosphonates, for producing a transdermal therapeutic system for treating osteoporosis.

## Revendications

1. Système thérapeutique transdermique pour le traitement de l'ostéoporose, **caractérisé en ce qu'**il contient de l'estriol en combinaison avec au moins une substance active de la classe des bisphosphonates.

2. Système thérapeutique transdermique selon la revendication 1, **caractérisé en ce qu'**il libère en 24 heures 8 à 16 mg d'estriol et 3 à 7 mg de bisphosphonate.

3. Utilisation d'estriol en combinaison avec au moins une substance active du groupe des bisphosphonates pour la préparation d'un système thérapeutique transdermique pour le traitement de l'ostéoporose.
